# EUROPEAN PATENT APPLICATION

(11) **EP 4 821 952 A1**
(43) Date of publication of application: **14.10.2026**
(21) Application number: 24902880.4
(22) Date of filing: 12.12.2024
(51) Int. Cl.: C07K 16/46, C07K 16/32, C12N 15/13, A61K 39/395, A61P 35/00

(54) **ANTI-HER2 BISPECIFIC ANTIBODY AND USE THEREOF**

(30) Priority: 12.12.2023 CN 202311705319; 08.10.2024 CN 202411401471
(71) Applicant: Shanghai Bao Pharmaceuticals Co., Ltd., Shanghai 201908 (CN)
(72) Inventor: LIU, Yanjun, Shanghai 201908 (CN); WANG, Zheng, Shanghai 201908 (CN); XU, Yunxia, Shanghai 201908 (CN)
(74) Representative: Bayramoglu et al.
(86) International application number: PCT/CN2024/138695
(87) International publication number: WO 2025/124463

(57) **Abstract**

Disclosed in the present disclosure is a stable anti-HER2 bispecific antibody, which comprises a first protein functional region and a second protein functional region used for recognizing extracellular domain II and extracellular domain IV of HER2, respectively. The bispecific antibody remains stable after being stored in PBS at about 37 °C for 30 days. Further disclosed in the present disclosure are a pharmaceutical composition comprising the bispecific antibody and a PD1 antibody, and the use thereof. The bispecific antibody and the pharmaceutical composition containing same of the present disclosure can be used for treating cancers.

## Description

### PRIORITY CLAIM

**T**he present disclosure claims priority to Chinese Patent Application No. 202311705319.X filed on December 12, 2023, and to Chinese Patent Application No. 2024114014713 filed on October 08, 2024, which are incorporated herein by reference in their entirety.

### TECHNICAL FIELD

The present disclosure relates to the field of biomedicine, and in particular to anti-HER2 bispecific antibodies and uses thereof.

### BACKGROUND TECHNOLOGY

Trastuzumab and pertuzumab, as anti-HER2 antibodies, are widely known for the treatment of cancers such as HER2-positive breast cancer. The combination of pertuzumab and trastuzumab demonstrates significantly greater efficacy compared to monotherapy. The response rate for pertuzumab monotherapy is only 3.4%, but the response rate for the combined use of pertuzumab and trastuzumab can reach 21.4%. Nevertheless, both trastuzumab and pertuzumab are applicable only to the treatment of HER2-highly-positive breast cancer (IHC test result of 3+). For HER2-low-positive breast cancer, the therapeutic effects of both trastuzumab and pertuzumab are not ideal. In recent years, cell therapy has achieved remarkable success in the treatment of hematologic tumors. Therefore, some scientists have developed a treatment based on trastuzumab for HER2-positive breast cancer. However, a patient died on day 5 after CAR-T cell infusion due to severe side effects (Morgan et al., The American Society of Gene & Cell Therapy 2010; 4:843-851). Bispecific antibodies targeting different epitopes of HER2 are monoclonal antibody drugs designed to recognize two distinct antigenic epitopes on the HER2 surface. They consist of two heavy chain variable regions from different antibodies and two identical light chain variable regions (CN105829347A; CN105820251A; CN105980409A), and they can specifically bind to the second and fourth domains of HER2. The aim is to provide a bispecific antibody that combines the activities of both trastuzumab and pertuzumab, replacing the current clinical treatment approach of combining the two antibodies of trastuzumab and pertuzumab. However, antibodies in the prior art suffer from poor antigen-binding activity or inadequate stability, often leading to precipitation. Stability is a prerequisite for the druggability of antibodies. Meanwhile, how to improve the overall efficacy of drugs remains an important requirement for drug research.

### CONTENT OF THE INVENTION

In view of the technical defects in the prior art, such as low affinity, poor stability, easy formation of precipitates, and susceptibility to degradation, the present disclosure aims to provide an anti-HER2 bispecific antibody and use thereof.

The inventors of the present disclosure have discovered that by utilizing the heavy chains of pertuzumab and trastuzumab, respectively, and optimizing the natural trastuzumab light chain as a common light chain, a bispecific antibody can be obtained, and this bispecific antibody exhibits affinity for the second and fourth domains of the HER2 target comparable to that of wild-type pertuzumab and trastuzumab, respectively. Meanwhile, it is unexpectedly discovered that compared to the anti-HER2 bispecific antibody molecule KJ015-C whose stability has been significantly improved relative to the unmutated sequence, the antibody KJ015-H disclosed herein demonstrates even more outstanding stability, shows significantly improved stability after thermal stability testing, and exhibits enhanced proliferation inhibitory effects and ADCC activity against various HER2-expressing tumor cells in further studies.

The antibody of the present disclosure, when used in combination with a programmed death receptor 1 (PD1) antibody, displays significantly superior tumor inhibitory activity compared to the combination of trastuzumab and pertuzumab.

In order to solve the above technical problems, embodiments of some aspects of the present disclosure provide an anti-HER2 bispecific antibody, and the bispecific antibody includes a first protein functional region and a second protein functional region for recognizing extracellular domain II and extracellular domain IV of HER2, respectively. The first protein functional region includes a first heavy chain and a common light chain, and the second protein functional region includes a second heavy chain and the common light chain; the bispecific antibody remains stable after storage in a PBS buffer at 37 °C±0.5 °C for 10, 20, 22, 24, 26, 28, or 30 days; optionally, the bispecific antibody shows an increase in the acidic peak proportion of less than 10%, less than 9%, less than 8%, less than 7%, less than 6%, or less than 5% after storage in a PBS buffer at 37 °C for 10, 20, 22, 24, 26, 28, or 30 days.

In some embodiments, the bispecific antibody of the present disclosure shows an increase in the acidic peak proportion of less than 5%, less than 4%, less than 3%, less than 2%, or less than 1% after storage in a PBS buffer for 10, 20, 22, 24, 26, 28, or 30 days, as detected by IEC-HPLC.

In some embodiments, the bispecific antibody of the present disclosure shows a decrease in the main peak proportion of less than 5%, less than 4%, less than 3%, less than 2.5%, or less than 2% after storage in a PBS buffer for 10, 20, 22, 24, 26, 28, or 30 days, as detected by IEC-HPLC.

In some embodiments, the bispecific antibody of the present disclosure includes a first protein functional region and a second protein functional region for recognizing extracellular domain II and extracellular domain IV of HER2, respectively. The first protein functional region includes a first heavy chain and a common light chain, and the second protein functional region includes a second heavy chain and the common light chain. The common light chain includes a light chain variable region having the amino acid sequence set forth in SEQ ID NO: 1, the first heavy chain includes a first heavy chain variable region having the amino acid sequence set forth in SEQ ID NO: 2 or a variant thereof, and the second heavy chain includes a second heavy chain variable region having the amino acid sequence set forth in SEQ ID NO: 5 or a variant thereof.

In some embodiments, the variant of the bispecific antibody of the present disclosure provides the bispecific antibody with stronger stability or tumor inhibitory activity.

In some embodiments, the bispecific antibody of the present disclosure further includes a light chain constant region. The light chain constant region is preferably a human light chain constant region λ chain or κ chain; preferably, the amino acid sequence of the light chain constant region is set forth in SEQ ID NO: 6.

In some embodiments, the first heavy chain and the second heavy chain of the bispecific antibody of the present disclosure further include heavy chain constant regions. The heavy chain constant regions are preferably human heavy chain constant regions; preferably, a heterodimer is formed between the first heavy chain and the second heavy chain of the bispecific antibody, and the first heavy chain and the second heavy chain are preferably linked via a Knob-in-Hole structure; more preferably, the heavy chain constant regions of the first heavy chain and the second heavy chain are set forth in SEQ ID NOs: 7 and 8, respectively.

In some embodiments, the bispecific antibody of the present disclosure has a reduced fucose modification; optionally, the antibody has no fucose modification.

Embodiments of other aspects include providing a nucleic acid molecule encoding the bispecific antibody described in the present disclosure.

Embodiments of some aspects include providing an expression vector, which includes a nucleotide sequence of the nucleic acid molecule described in the present disclosure.

Embodiments of some aspects include providing a prokaryotic or eukaryotic cell, which includes the expression vector described in the present disclosure.

Embodiments of some aspects include providing a drug conjugate of the anti-HER2 bispecific antibody, which is obtained by covalent conjugation of the bispecific antibody as described above to a cytotoxic drug.

Embodiments of some aspects include providing a pharmaceutical composition, which includes the bispecific antibody of the present disclosure or the drug conjugate of the bispecific antibody of the present disclosure; optionally, the pharmaceutical composition further includes a hyaluronidase and/or an anti-PD1 antibody; optionally, the hyaluronidase is rHuPH20 or modified rHuPH20; meanwhile, optionally, the anti-PD1 antibody is selected from pembrolizumab; optionally, the pharmaceutical composition further includes a pharmaceutically acceptable carrier or excipient.

Embodiments of some aspects include providing a method for preparing the bispecific antibody described in the present disclosure, which includes the following steps:
(1) constructing nucleotide sequences which include a nucleotide sequence encoding the first heavy chain,a nucleotide sequence encoding the second heavy chain, and a nucleotide sequence encoding the common light chain in the same vector or different vectors; and
(2) expressing the vector(s) constructed in the above step in different host cells or the same host cell.

Embodiments of some aspects include providing use of the anti-HER2 bispecific antibody described in the present disclosure, the drug conjugate of the anti-HER2 bispecific antibody described in the present disclosure, and/or the pharmaceutical composition described in the present disclosure in preparing a medicament for treating an HER2-positive tumor; optionally, the HER2-positive tumor is selected from breast cancer, gallbladder carcinoma, gastric cancer, and lung cancer.

In addition, in order to solve the above technical problems, the technical solution of yet another aspect is to provide a kit combination, which includes kit A and kit B. Kit A includes the anti-HER2 bispecific antibody, the drug conjugate of the anti-HER2 bispecific antibody, or the pharmaceutical composition including the bispecific antibody of the present disclosure, and kit B includes another antibody targeting HER2 or other targets (e.g., an antibody targeting PD1), a bispecific antibody, a genetically modified cell, or a pharmaceutical composition. Kit A and kit B are used in no particular order. For example, kit A is used before kit B, kit B is used before kit A, or kit A and kit B are used simultaneously.

The anti-HER2 bispecific antibody, the immunoconjugate, the pharmaceutical composition, or the kit combination described in the present disclosure can be administered to a patient for treating a related tumor.

In the present disclosure, the anti-HER2 bispecific antibody, an antigen-binding moiety thereof, a mixture thereof, or a therapeutic immune cell including the antigen-binding moiety thereof may also be used in combination with a chemotherapeutic drug and/or an additional antibody. Therefore, the composition of the present disclosure may also include a chemotherapeutic drug and/or an additional antibody.

In the present disclosure, the chemotherapeutic drug includes, but is not limited to: adriamycin, cyclophosphamide and taxanes (paclitaxel and docetaxel), capecitabine, gemcitabine, vinorelbine, tamoxifen, aromatase inhibitors [Arimidex (anastrozole), Femara (letrozole), Aromasin (exemestane)], 5-fluorouracil (5-FU) + folinic acid, irinotecan, oxaliplatin, cisplatin, carboplatin, estramustine, mitoxantrone, prednisone, vincristine, etc., or combinations thereof.

### DESCRIPTION OF THE DRAWINGS

FIG**.** 1 shows the detection results of the proliferation inhibitory effects of different anti-HER2 bispecific antibodies in three different types of tumor cells.
FIG. 2 shows the detection results of the ADCC effects of different anti-HER2 bispecific antibodies. The ADCC activity of the samples before and after storage at 37 °C for 30 days was detected. The ADCC activity of KJ015-C was set as 100% to evaluate the activity of KJ015-D and KJ015-H, in which FIG. 2a shows the evaluation of the activity of the antibodies before heating and storage, and FIG. 2b shows the evaluation of the activity of the antibodies after heating and storage.
FIG. 3 shows the results of the stability of KJ015-H tested by different methods. FIG. 3a shows the investigation of the stability of KJ015-H after storage at 37 °C for 30 days using IEC-HPLC, and FIG. 3b shows the investigation of the stability of KJ015-H after storage at 37 °C for 30 days using SEC-HPLC.
FIG. 4 shows that KJ015-H can reduce the viability of HER2-expressing cancer cell strains and inhibit signal transduction downstream of HER2. FIGs. 4a-d show the measurement of the viability in Calu-3 (a), NCI-N87 (b), MDA-MB-176 (c), and BT474 (d) cells using CCK-8, respectively.
FIG. 5 shows the detection results of the tumor growth inhibition ability of the HER2 antibody samples of each group in human tumor cell line-derived xenograft (CDX) models. FIGs. 5a-5d show the tumor growth curves of xenografts derived from BT474 (Her2 3+, breast cancer), NCI-N87 (Her2 3+, gastric cancer), MDA-MB-175 (Her2 1+, breast cancer), and Calu-3 (Her2 3+, lung adenocarcinoma) cell lines in mice, respectively.
FIG. 6 shows the detection results of the tumor growth inhibition ability of the HER2 antibody samples of each group in a gallbladder carcinoma (GBC) patient-derived xenograft (PDX) model, in which T+P refers to the combined use of trastuzumab + pertuzumab.
FIG. 7 shows the results of the effects of the HER2 antibody samples of each group in a PBMC-humanized NCI-N87 xenograft model. FIG. 7a shows the tumor growth in the PBMC-humanized NCI-N87 xenograft model after different treatments; FIG. 7b shows the comparison of tumor sizes across groups on d18 after administration.
FIG. 8 shows the localization coordinates of the HER2 molecule on the surface of antibody-treated SK-BR-3 cells determined by stochastic optical reconstruction microscopy.
FIG. 9 shows the results of the immunoblotting experiment in Example 12.

### DETAILED DESCRIPTION OF THE INVENTION

### Definition

Unless otherwise defined, all technical and scientific terms used herein have the same meaning as commonly understood by those of ordinary skill in the art. For the purposes of the present disclosure, the following terms are defined below.

The term "about" used in combination with a numerical value is intended to encompass the numerical values in a range from a lower limit less than the specified numerical value by 5% to an upper limit greater than the specified numerical value by 5%.

The term "and/or" should be understood to refer to any one of the options or a combination of any two or more of the options.

As used herein, the term "comprise" or "include" is intended to mean that the described elements, integers, or steps are included, but not to the exclusion of any other elements, integers, or steps. As used herein, the term "comprise" or "include", unless otherwise indicated, also encompasses the situation where the entirety consists of the described elements, integers, or steps. For example, when referring to an antibody variable region "including" a specific sequence, the term is also intended to encompass an antibody variable region consisting of the specific sequence.

As used herein, the term "antibody" refers to a polypeptide including at least an immunoglobulin light chain variable region or heavy chain variable region that specifically recognizes and binds to an antigen. This term encompasses a variety of antibody structures, including but not limited to, monoclonal antibodies, polyclonal antibodies, single-chain or multi-chain antibodies, monospecific or multispecific antibodies (e.g., bispecific antibodies), fully human or chimeric or humanized antibodies, full-length antibodies, and antibody fragments, so long as they exhibit the desired antigen-binding activity.

The term "antigen-binding fragment" (used interchangeably herein with "antibody fragment" and "antigen-binding moiety") of an antibody refers to a molecule that is not an intact antibody but contains a portion of an intact antibody responsible for binding to an antigen to which the intact antibody would bind. As will be appreciated by those skilled in the art, the antigen-binding moiety of an antibody generally includes amino acid residues from a "complementarity determining region" or "CDR". The antigen-binding fragment may be prepared by recombinant DNA techniques, or by enzymatic or chemical cleavage of an intact antibody. The antigen-binding fragment includes, but is not limited to, Fab, scFab, Fab', F(ab')2, Fab'-SH, Fv, single-chain Fv, diabody, triabody, tetrabody, minibody, and single-domain antibody (sdAb). For a more detailed description of the antibody fragment, see*:* Fundamental Immunology, W.E. Paul (ed.), Raven Press, N.Y. (1993); Shao Rongguang et al. (eds.), Antibody Drug Research and Application, People's Medical Publishing House (2013); Hollinger et al., PNAS USA 90:6444-6448 (1993); Hudson et al., Nat. Med. 9:129-134 (2003).

The term "variable region" or "variable domain" refers to a domain of a heavy chain or light chain of an antibody involved in the binding of the antibody to an antigen. Variable domains of heavy and light chains of natural antibodies typically have similar structures, where each domain includes four c*on*served framework regions (FRs) and three complementarity determining regions (see, e.g., Kindt et al., *Kuby Immunology*, 6th Edition, W. H. Freeman and Co., p. 91 (2007)). A single VH or VL domain can be sufficient to provide antigen-binding specificity. In addition, a VH or VL domain from an antibody that binds to a particular antigen can be used to isolate antibodies that bind to the antigen, enabling the screening of libraries of complementary VL or VH domains, respectively. See, e.g., Portolano et al., J. Immunol.150: 880-887 (1993); Clarkson et al., Nature, 352: 624-628 (1991).

"Variant" refers to a new peptide fragment or a fragment obtained by adding or reducing amino acid residues or replacing other amino acid residues on the basis of the amino acid sequence of a certain peptide fragment or a fragment thereof.

Variable regions generally exhibit the same general structure of relatively conserved framework regions (FRs) connected by three hypervariable regions, which are also known as complementarity determining regions or CDRs. CDRs from the two chains of each pair are generally aligned by the framework regions, such that the CDRs can bind to specific epitopes. The variable regions of both the light and heavy chains, from the N-terminus to the C-terminus, generally include domains FR1, CDR1, FR2, CDR2, FR3, CDR3, and FR4.

"Complementarity determining region" or "CDR region" or "CDR" or "hypervariable region" (used interchangeably herein with hypervariable region "HVR") is a region in an antibody variable domain that is highly variable in sequence and forms a structurally defined loop ("hypervariable loop") and/or includes antigen-contacting residues ("antigen-contacting sites"). The CDRs are primarily responsible for binding to antigenic epitopes. The CDRs of heavy and light chains are generally referred to as CDR1, CDR2, and CDR3, and are numbered sequentially from the N-terminus. The CDRs located in a heavy chain variable domain of an antibody are referred to as HCDR1, HCDR2, and HCDR3, whereas the CDRs located in a light chain variable domain of an antibody are referred to as LCDR1, LCDR2, and LCDR3. In a given amino acid sequence of a light chain variable region or a heavy chain variable region, the exact amino acid sequence boundary of each CDR can be determined using any one or a combination of many well-known antibody CDR assignment systems, including, for example, Chothia based on the three-dimensional structure of antibodies and the topology of CDR loops, Kabat based on antibody sequence variability, AbM (University of Bath), Contact (University College London), and the international ImMunoGeneTics database (IMGT). The Chothia CDR definition is the same as the Kabat CDR definition, except for HCDR1 and HCDR2. The North CDR definition is based on affinity propagation clustering using a large number of crystal structures.

The term "prevention" includes the inhibition or delay of the onset or onset frequency of a disease or disorder or symptoms thereof, and generally refers to the administration of a drug prior to the onset of a disorder or symptoms, particularly in individuals at risk.

As used herein, the term "antibody stability" refers to the change in activity of an antibody after storage at a specific temperature and time. Applicable methods for stability testing include assessing the distribution of charge variants by cation-exchange chromatography using the IEC-HPLC method (other commonly used testing methods for antibody stability may be supplemented as appropriate). Preferably, with reference to molecular exclusion chromatography (Chinese Pharmacopoeia 2010 Edition, Volume III, Appendix IIIB), the HER2 bispecific antibodies are tested, and the thermal stability testing data of the antibodies before and after storage in a PBS system at 37 °C and pH 7.4 for 30 days are compared. The stability of the bispecific antibodies is expressed using the change (increase or decrease) proportion of the acidic peak, the main peak, or the basic peak as an indicator. Increase proportion of acidic/basic peak = [(percentage of acidic/basic peak after 30 days of storage - percentage of acidic/basic peak before storage) × 100%]/percentage of acidic/basic peak before storage; decrease proportion of main peak = [(percentage of main peak before storage - percentage of main peak after 30 days of storage) × 100%]/percentage of main peak before storage. "Stable" means that after the bispecific antibody is stored in a PBS buffer at 37 °C±0.5 °C for 10, 20, 22, 24, 26, 28, or 30 days, the acidic peak proportion does not increase or increases by less than 5%, less than 4%, less than 3%, less than 2%, less than 1%, or less than 0.5%; and/or the main peak proportion does not decrease or decreases by less than 5%, less than 4%, less than 3%, less than 2.5%, or less than 2%, as detected by IEC-HPLC.

"Pharmaceutical composition" refers to a formulation or a combination of formulations including one, two, or more active ingredients, which allows the active ingredients contained therein to be present in an effective and bioactive form. The "pharmaceutical composition", when present in the form of a combination of separate formulations containing two or more different active ingredients, can be administered simultaneously, sequentially, separately, or intermittently, with the purpose of exerting the bioactivity of the multiple active ingredients for jointly treating the disease.

"Effective amount" is generally an amount sufficient to reduce the severity and/or frequency of symptoms, eliminate these symptoms and/or underlying causes, prevent the appearance of symptoms and/or underlying causes thereof, and/or ameliorate or improve damage caused by or associated with a disease state (e.g., lung disease). In some embodiments, the effective amount is a therapeutically effective amount or a prophylactically effective amount. "Therapeutically effective amount" is an amount sufficient to treat a disease state or symptoms, particularly a state or symptoms associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur by administration of one dose, and may occur only after administration of a series of doses. Thus, a therapeutically or prophylactically effective amount may be administered in one or more administrations.

The terms "host cell", "host cell line", and "host cell culture" are used interchangeably and refer to cells into which exogenous nucleic acids are introduced, including progenies of such cells. Host cells include "transformants" and "transformed cells", which include primary transformed cells and progenies derived therefrom, regardless of the number of passages. Progenies may not be exactly the same as parent cells in terms of nucleic acid content, and may contain mutations. Mutant progenies that have the same function or bioactivity as screened or selected from the initially transformed cells are included herein.

"Transfection" refers to the introduction of an exogenous nucleic acid into a eukaryotic cell. Transfection may be accomplished by a variety of means known in the art, including electroporation, microinjection, liposome fusion, and the like.

"Nucleic acid molecule encoding", "coding DNA sequence", and "coding DNA" refer to the order of deoxyribonucleotides along a deoxyribonucleic acid strand. The order of these deoxyribonucleotides determines the order of the amino acids along the polypeptide (protein) chain. Thus, a nucleic acid sequence encodes an amino acid sequence.

As used herein, the term "tumor" refers to a disease characterized by the pathological proliferation of cells or tissues, and their subsequent migration or invasion of other tissues or organs. Tumor growth is generally uncontrolled and progressive and does not induce or inhibit normal cell proliferation. Tumors include "cancers", which generally refer to all malignant tumors.

"Treatment" refers to a clinical intervention in an attempt to alter the course of a disease in an individual or to treat a cell-caused disease, which may be applied for prevention or during the clinical pathological course. Therapeutic effects include, but are not limited to, preventing the onset or recurrence of a disease, alleviating symptoms, reducing any direct or indirect pathological consequences of any disease, preventing metastasis, slowing the rate of disease progression, ameliorating or relieving the disease condition, relieving or improving the prognosis, and the like.

"Combination" relates to a treatment regimen that provides at least two or more different therapies to achieve a specified therapeutic effect. The therapies may be physical, such as radiotherapy, or chemical, such as a drug administered to a subject, which also includes a combination drug. "Combination drug" refers to a combination including two or more pharmaceutical formulations each having an active ingredient, which require combined use when administered to a subject. The active ingredients may be mixed together to form a single administration unit or may remain separate as independent administration units for separate use; when administered, different pharmaceutical formulations may be administered substantially synchronously, simultaneously, or sequentially.

The term "pharmaceutically acceptable" refers to those compounds, materials, compositions, and/or dosage forms that are suitable for use in contact with human and animal tissues without excessive toxicity, irritation, allergic response, or other problems or complications, commensurate with a reasonable benefit/risk ratio.

As used herein, the "anti-PD1 antibody" or "PD1 antibody" refers to an antibody or an antigen-binding fragment thereof that targets the immune checkpoint protein PD1 (also known as PD-1) and is capable of blocking or affecting the binding of PD1 to ligands thereof. The scope of selectable anti-PD1 antibodies includes, but is not limited to, pembrolizumab. "programmed death receptor 1", "programmed cell death protein 1", or "PD1", also known as CD279, belongs to the immunoglobulin superfamily.

On the basis of the general knowledge in the art, the above optional conditions can be combined arbitrarily to obtain preferred embodiments of the present disclosure. The reagents and starting materials used in the present disclosure are commercially available.

The positive and progressive effects of the present disclosure are as follows: a bispecific antibody is formed by mutation of designated amino acids in the light chain variable region of trastuzumab, the heavy chain variable region of pertuzumab, and the heavy chain variable region of trastuzumab, and this results in a bispecific monoclonal antibody with high affinity, good stability, low susceptibility to degradation, potent tumor cell-killing activity, and high ADCC activity, as well as an anti-PD1 antibody and/or hyaluronidase for use in combination with the bispecific antibody.

### SPECIFIC IMPLEMENTATIONS

The technical solutions of the present disclosure are further illustrated with reference to the following specific examples. It will be appreciated by those skilled in the art that the examples are only intended to help understand the present disclosure and should not be construed as specific limitations to the present disclosure.

Experimental procedures without specified conditions in the following examples were performed in accordance with conventional procedures and conditions, or in accordance with product instructions.

### Example 1. Antibody Construction, Expression, and Preparation

According to the amino acid sequences in Table 2, gene synthesis of the antibody light and heavy chains was performed according to Table 1, and plasmid extraction was performed to obtain a corresponding common light chain plasmid, a first heavy chain plasmid, and a second heavy chain plasmid. The plasmid DNA containing the coding sequences of the first heavy chain, the second heavy chain, and the common light chain described above was co-transfected into an ExpiCHO-S cell strain (Gibco) at a certain ratio (1:1:2) using a Rhinogen^{®} transient transfection kit. The procedures were performed according to the instructions of the Rhinogen^{®} transfection kit. Finally, cell strains expressing various bispecific antibodies were obtained. Each cell strain was expanded to a volume of 100 mL using a CD2 basal medium and subjected to fed-batch culture. Starting from day 3, 2% Feed4 was added daily. After 12 days of suspension culture, the culture supernatants were separately collected and purified by Protein A affinity chromatography to obtain bispecific monoclonal antibody protein samples. The purified samples were sterilized, filtered through a 0.22 µm membrane, and stored at 2-8 °C.

**Table 1. Sequence combinations of HER2 bispecific antibodies**

| Name | Common light chain | First heavy chain | Second heavy chain |
|---|---|---|---|
| KJ015-C | | | HER2 antibody second heavy chain-C |
| KJ015-D | Her2 antibody common light chain | HER2 antibody first heavy chain | HER2 antibody second heavy chain-D |
| KJ015-H | | | HER2 antibody second heavy chain-H |

*The Her2 antibody common light chain was formed by directly linking the Her2 antibody common light chain variable region to the HER2 antibody light chain constant region; the Her2 antibody first heavy chain was formed by directly linking the Her2 antibody first heavy chain variable region to the HER2 antibody first heavy chain constant region; the Her2 antibody second heavy chain was formed by directly linking the corresponding Her2 antibody second heavy chain variable region to the HER2 antibody second heavy chain constant region, i.e., the Her2 antibody second heavy chain-H was formed by directly linking the Her2 antibody second heavy chain variable region-H (SEQ ID NO: 5) to the HER2 antibody second heavy chain constant region (SEQ ID NO: 8). The same applies to the others (see Table 2 for specific sequences). HER2 and Her2 are used interchangeable.

**Table 2. Amino acid sequences of HER2 bispecific antibody fragments**

| **Name** | **Sequence** |
|---|---|
| HER2 antibody common light chain variable region (SEQ ID NO: 1) | DIQMTQSPSSLSASVGDRVTITCRASQDVSTAVAWYQQKPGKAPKLLIY SASYLYSGVPSRFSGSRSGTDFTLTISSLQPEDFATYYCQQHYTTPPTFGQGTK VEIK |
| HER2 antibody first heavy chain variable region (SEQ ID NO: 2) | EVQLVESGGGLVQPGGSLRLSCAASGFNIKDTYIHWVRQAPGKGLEWV ARIYPTNGYTRYADSVKGRFTISADTSKNTAYLQMNSLRAEDTAVYYCSRW GGEGFYAMDYWGQGTLVTVSS |
| HER2 antibody second heavy chain variable region-C (SEQ ID NO: 3) | EVQLVESGGGLVQPGGSLRLSCAASGFTFTDYTMDWVRQAPGKGLEW VADVNPNSGGSIYNQRFKGRFTLSVDRSKNTLYLQMNSLRAEDTAVYYCAR NLGPWFYFDYWGQGTLVTVSS |
| HER2 antibody second heavy chain variable region-D (SEQ ID NO: 4) | EVQLVESGGGLVQPGGSLRLSCAASGFTFTDYTMDWVRQAPGKGLEW VADVNPNSGGSILNRRFKGRFTLSVDRSKNTLYLQMNSLRAEDTAVYYCAR NLGPNFYFDYWGQGTLVTVSS |
| HER2 antibody second heavy chain variable region-H (SEQ ID NO: 5) | EVQLVESGGGLVQPGGSLRLSCAASGFTFTDYTMDWVRQAPGKGLEW VADVNPNSGGSILNRRFKGRFTLSVDRSKNTLYLQMNSLRAEDTAVYYCAR NLGPLFYFDYWGQGTLVTVSS |
| HER2 antibody light chain constant region (SEQ ID NO: 6) | |
| HER2 antibody first heavy chain constant region (SEQ ID NO: 7) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKC KVSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFSC SVMHEALHNHYTQKSLSLSPGK |
| HER2 antibody second heavy chain constant region (SEQ ID NO: 8) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKC KVSNKALPAPIEKTISKAKGQPREPQVCTLPPSREEMTKNQVSLSCAVKGFYP SDIAVEWESNGQPENNYKTTPPVLDSDGSFFLVSKLTVDKSRWQQGNVFSCS VMHEALHNHYTQKSLSLSPGK |
| HER2 antibody first heavy chain constant region (SEQ ID NO: 9) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKC KVSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFSC SVMHEALHNHYTQKSLSLSPG |
| HER2 antibody first heavy chain constant region (SEQ ID NO: 10) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKC KVSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFSC SVMHEALHNHYTQKSLSLSP |
| HER2 antibody second heavy chain constant region (SEQ ID NO: 11) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKC KVSNKALPAPIEKTISKAKGQPREPQVCTLPPSREEMTKNQVSLSCAVKGFYP SDIAVEWESNGQPENNYKTTPPVLDSDGSFFLVSKLTVDKSRWQQGNVFSCS VMHEALHNHYTQKSLSLSPG |
| HER2 antibody second heavy chain constant region (SEQ ID NO: 12) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKC KVSNKALPAPIEKTISKAKGQPREPQVCTLPPSREEMTKNQVSLSCAVKGFYP |
| | SDIAVEWESNGQPENNYKTTPPVLDSDGSFFLVSKLTVDKSRWQQGNVFSCS VMHEALHNHYTQKSLSLSP |

### Example 2. Stability Testing

Distribution of charge variants was assessed by cation-exchange chromatography using the IEC-HPLC method. With reference to molecular exclusion chromatography (Chinese Pharmacopoeia 2010 Edition, Volume III, Appendix IIIB), the HER2 bispecific antibodies KJ015-C, KJ015-D, and KJ015-H were tested, and the thermal stability testing data of the antibodies before and after storage in a PBS system at 37 °C and pH 7.4 for 30 days were compared.

Dionex ProPac WCX-10, 4 × 250 mm, was used as a chromatographic column, and a mixed solution of 4 mM imidazole, 4 mM piperazine, 4 mM Tris, and 0.5 M NaCl was used as the mobile phase. The flow rate was 1 mL per minute, and the detection wavelength was 280 nm. 10 µL of the test sample solution was injected into a liquid chromatograph for testing.

According to the charge heterogeneity detected by IEC, the KJ015-H sample showed the least decrease in the main peak after heating, indicating greater stability (Table 3).

**Table 3. Charge heterogeneity detected by IEC**

| Sample name | Acidic peak content (%) | Acidic peak increase proportion (%) | Main peak content (%) | Main peak decrease proportion (%) | Basic peak content (%) |
|---|---|---|---|---|---|
| KJ015-C | 17.02 | 6.31 | 67.96 | 8.09 | 15.00 |
| KJ015-C-thermostable | 23.33 | | 59.87 | | 16.78 |
| KJ015-D | 3.11 | 6.98 | 88.23 | 8.35 | 8.66 |
| KJ015-D-thermostable | 10.09 | | 79.88 | | 10.04 |
| KJ015-H | 2.19 | 0.41 | 84.35 | 1.84 | 13.47 |
| KJ015-H-thermostable | 2.60 | | 82.51 | | 14.91 |

### Example 3. Detection of Proliferation Inhibitory Effects of Different HER2 Bispecific Antibodies

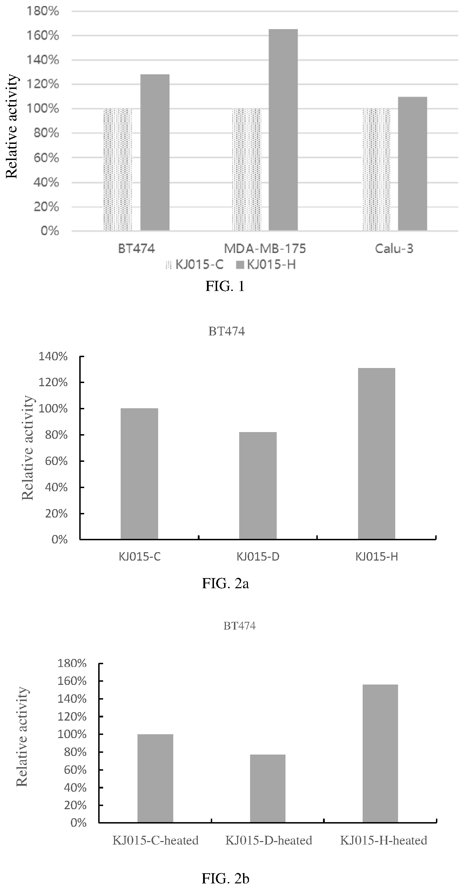

Different tumor cells were taken for subculture, diluted to different concentrations, and incubated with antibodies, with the day of addition counted as day 0. On the fourth day after addition, the detection plate was taken out from the incubator, 20 µL of CCK-8 staining solution was added to each well, and staining was performed in an incubator at 37 °C for 6 h, followed by reading on a microplate reader at 450 nm/630 nm. SoftMax Pro data processing software was used for automatic analysis, with the concentrations of the reference and test samples as the horizontal coordinate and the OD value as the vertical coordinate, and the fourparameter method was selected to fit the dose-response curves of the reference and test samples. The KJ015-C sample was used as the standard and defined as 100%, and the relative activity of other samples was calculated. The detection results show that the proliferation inhibitory activity of KJ015-H in different tumor cells was stronger than that of KJ015-C, with the proliferation inhibitory potency in BT474, MDA-MB-175, and Calu-3 cells being increased by 1.25-fold, 1.7-fold, and 1.1-fold, respectively, compared to KJ015-C (FIG. 1).

### Example 4. Detection of ADCC Effects of Different HER2 Bispecific Antibodies

In the ADCC detection, according to the cell growth rate, tumor cells in the logarithmic growth phase were resuspended in a medium and seeded into a 96-well plate. After adhesion, the antibodies KJ015-C, KJ015-D, KJ015-H, KJ015-C-heated, KJ015-D-heated, and KJ015-H-heated were added, and the plate was incubated at 37 °C with 5% CO₂ for 30 min. ADCC was assayed using the CytoTox96 non-radioactive cytotoxicity assay. The KJ015-C and KJ015-C-heated samples were used as the standards and defined as 100%, and the relative activity of other samples was calculated. The detection shows that on BT474 cells, the ADCC results indicate that the relative activity of KJ015-H was higher than that of KJ015-C and KJ015-D; the ADCC activity of the heated sample KJ015-H-heated was higher than that of the heated sample KJ015-Cheated, indicating that the ADCC capability of the KJ015-H molecule was stronger compared to the KJ015-C and KJ015-D molecules as well as the heated samples (see FIG. 2a and FIG. 2b).

### Example 5. Antibody Affinity Assay

The affinity of the direct interaction between KJ015-H, trastuzumab, pertuzumab, anbenitamab, zanidatamab and HER2 domains II and IV was detected by surface plasmon resonance (SPR) using a BIAcore^{®} 3000 instrument (Biacore, Uppsala, Sweden).

**Table 4. Kinetic constants and affinity constants of different antibodies for domains II and IV of HER2**

| **KD of sample (nM)** | KJ015 | Trastuzumab | Pertuzumab | Anbenitamab | Zanidatamab |
|---|---|---|---|---|---|
| **HER2 Domain II** | 5.24±0.34 | / | 2.26±0.13 | 25.4±0.6 | 0.69±0.06 |
| **HER2 Domain IV** | 0.63±0.07 | 0.90±0.09 | / | 0.25±0.06 | 2.25±0.51 |

The binding affinities of KJ015-H for Her2 domain II and domain IV were 5.24 nM and 0.63 nM, respectively (see Table 4). The affinity of pertuzumab for Her2 domain II was 2.26 nM, and the affinity of trastuzumab for domain IV was 0.90 nM. This indicates that KJ015-H possesses both the high affinity of trastuzumab and pertuzumab for Her2 domains II and IV.

### Example 6. Antibody Stability Testing

The thermal melting point (Tm) of each sample was measured using an Uncle instrument (Unchained Labs, Pleasanton, CA, USA).

SEC-HPLC analysis was performed under non-denaturing conditions using a Waters Acquity ARC system, and the purity was calculated using the area normalization method.

**Table 5. Tm of different antibodies KJ015-H, trastuzumab, and pertuzumab**

| **Sample name** | **Tm Onset (°C)** | **Tm1 (°C)** CH2/CH3 | **Tm2 (°C) Fab** | **Tm3 (°C) Fab2** |
|---|---|---|---|---|
| KJ015-H | 67.45 | 72.08 | 85.05 | 92.42 |
| Trastuzumab | 40.17 | 71.02 | 82.53 | - |
| Pertuzumab | 66.98 | 71.82 | 83.89 | - |

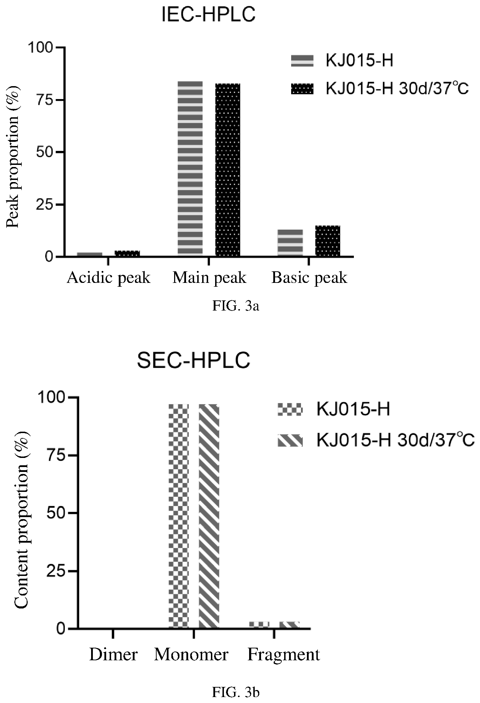

The sequence of KJ015-H is derived from trastuzumab and pertuzumab, and the melting temperature of KJ015-H is similar to that of pertuzumab (see Table 5). In terms of thermal stability, the engineering of KJ015-H significantly improved stability; even after storage in a PBS system at 37 °C for 30 days, no significant change was observed in either IEC purity or SEC purity (see FIG. 3a and FIG. 3b), whereas earlier studies have shown that trastuzumab undergoes severe degradation, with a significant drop in antigen-binding activity and bioactivity, after storage under the same conditions for 12 days (Schmid et al., 2018 Commun Biol 1, 28).

### Example 7. Inhibition of Cell Activity by KJ015-H

The viability of various cell lines was detected using the CCK-8 method. Cells were seeded into a 96-well plate. After cell attachment, cells were treated with antibodies (human IgG1, trastuzumab (T), pertuzumab (P), trastuzumab plus pertuzumab (T+P), KJ015-H, anbenitamab (Anbe), zanidatamab (Zani)) for 120 h. A CCK-8 solution was added to each well, and the cells were incubated for 1 h. Subsequently, the absorbance of each well was measured, and the results were subjected to statistical analysis.

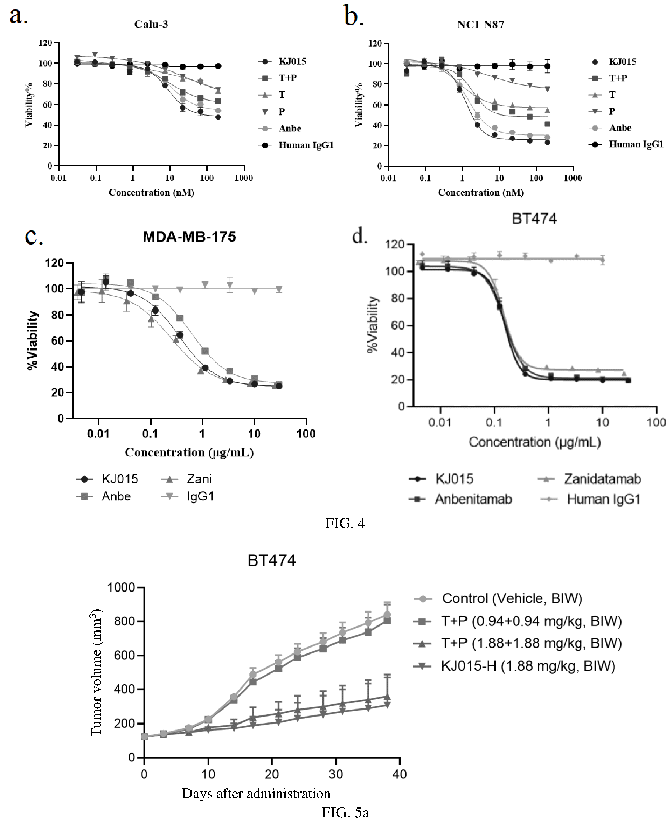

As shown in FIG. 4, in the four cell lines, as the concentration of KJ015-H increased, the inhibition rate also increased. KJ015-H showed strong cytotoxicity in all cell lines (FIGs. 4a-d). These results indicate that KJ015-H can reduce the viability of MDA-MB-175, BT474, Calu-3, and NCI-N87 cells and inhibit the proliferation of each type of tumor cells.

### Example 8. Anti-Tumor Effects of HER2 Antibody Samples of Each Group in CDX Models

NCI-N87, Calu-3, BT474, and MDA-MB-175 cells are estrogen-dependent in mice. Therefore, estradiol pellets were implanted into the right mammary fat pads of the mice. Subsequently, 1 × 10⁷ cells were injected subcutaneously into the right anterior dorsum of female nude mice to establish cancer xenograft models. For the NCI-N87, Calu-3, and BT474 xenograft models, when the mean tumor volume of hormonized mice reached about 80-150 mm³, the mice were randomized into 4 groups of 6 mice each according to the experimental design. For the MDA-MB-175 xenograft model, when the mean tumor volume of hormonized mice reached about 250 mm³, the mice were randomized into 5 groups of 6 mice each according to the experimental design.

The BT474 or NCI-N87 xenograft group was treated with vehicle, trastuzumab + pertuzumab (0.94+0.94 mg/kg, 1.88+1.88 mg/kg), or KJ015-H (1.88 mg/kg) via tail vein injection twice weekly.

The MDA-MB-175 xenograft group was treated with vehicle, trastuzumab + pertuzumab (1.5+1.5 mg/kg, 5+5 mg/kg), or KJ015-H (3 mg/kg, 10 mg/kg) via tail vein injection administration twice weekly.

Calu-3 xenograft mice were treated with vehicle, trastuzumab + pertuzumab (1.88+1.88 mg/kg, 3.75+3.75 mg/kg), or KJ015-H (3.75 mg/kg) via tail vein injection twice weekly. Tumor diameters and mouse body weights were measured twice weekly to calculate tumor volumes. The data are presented as mean ± SEM (n = 6).

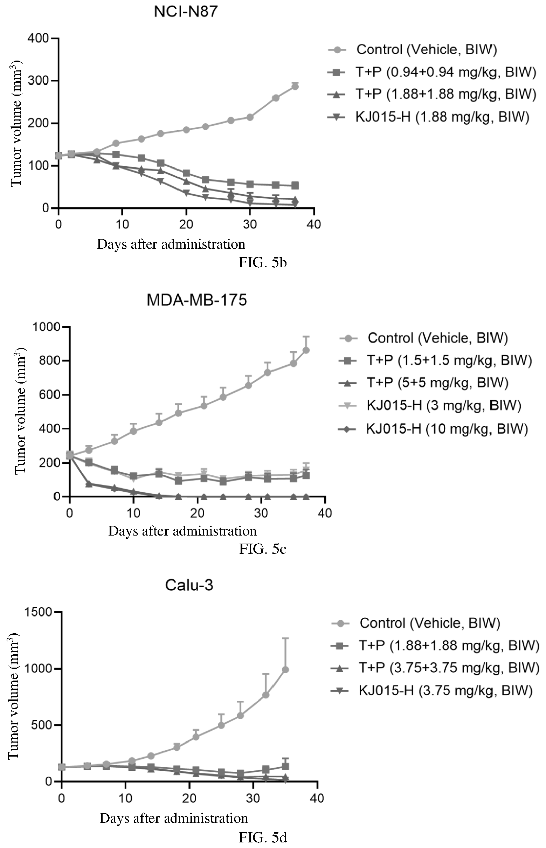

Throughout the treatment and observation period, KJ015-H could potently reduce tumor growth and showed a trend of being stronger than trastuzumab + pertuzumab (FIGs. 5a-5d). Particularly in the breast cancer BT474 cell xenograft model, compared to the control group, the low-concentration trastuzumab + pertuzumab combination (a molar concentration of 1:1, 0.94 mg/kg per antibody) had no effect on tumor size, but KJ015-H at the same concentration (1.88 mg/kg) still induced tumor regression. These results indicate that KJ015-H can significantly inhibit tumor growth at low concentrations without producing marked toxicity compared to trastuzumab + pertuzumab.

### Example 9. Detection of Anti-Tumor Effects of HER2 Antibody Samples of Each Group in PDX Model

In order to simplify the treatment regimen for tumor patients and reduce costs for both patients and the medical system, hyaluronidase was added to the KJ015-H injection by Shanghai Bao Pharmaceuticals Co., Ltd., changing the traditional intravenous injection of the antibody to subcutaneous injection. The KJ015-H subcutaneous injection (hyaluronidase-containing formulation) is a compound formulation of KJ015-H and hyaluronidase (Shanghai Bao Pharmaceuticals Co., Ltd.) used for subcutaneous injection. Hyaluronidase can temporarily degrade hyaluronic acid in the human body, improve the permeability of tissues, promote the rapid dispersion and absorption of the injected drug, and realize subcutaneous injection administration.

In this example, the *in vivo* anti-tumor activity of KJ015-H subcutaneous injection was investigated in a GBC patient-derived xenograft (PDX) mouse model. A piece of tumor tissue measuring 2 × 2 × 2 mm³ from GBC patient-derived xenografts (PDXs) was implanted subcutaneously in the right forelimb of 8-week-old C-NKG mice (severely immunodeficient mice generated by Cyagen through deletion of the Il2rg gene from NOD-Scid genetic background mice). When tumors reached an average of 130 mm³, 30 mice were randomized into 5 groups to receive intravenous injection of vehicle or trastuzumab + pertuzumab (12.5+12.5 mg/kg), or subcutaneous injection of KJ015-H (12.5 mg/kg, 25 mg/kg, 50 mg/kg), with tumor growth measured twice weekly. Throughout the investigation, mice were weighed and regularly observed for symptoms of disease or discomfort related to tumor growth and/or drug toxicity.

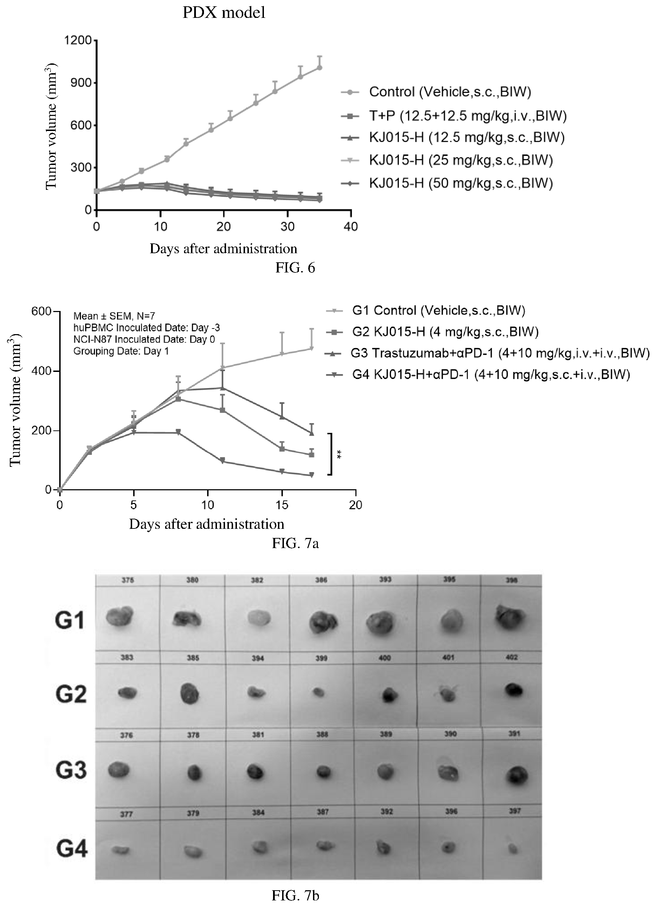

Compared to the control group, both the KJ015-H subcutaneous injection and trastuzumab + pertuzumab significantly inhibited tumor growth with no significant difference observed between these experimental groups, and no obvious toxicity was induced according to the body weight loss. The degree of tumor inhibition by the KJ015-H subcutaneous injection showed a dose-dependent trend. The results show that, consistent with the results of the *in vitro* and *in vivo* investigations described above, the KJ015-H subcutaneous injection had the same anti-tumor effect as high-concentration intravenous injection of trastuzumab + pertuzumab when injected subcutaneously at a low concentration (see FIG. 6 and Table 6).

**Table 6. Effect of KJ015-H subcutaneous injection on tumor weight in PDX model**

| **Group** | **Antibody sample** | **Tumor weight (mg)** | **T_{weight}/C_{weight} (%)** | **P** |
|---|---|---|---|---|
| **G1** | IgG1 | 1423.7±163.6 | -- | -- |
| **G2** | Trastuzumab + Pertuzumab (12.5 mg/kg for each antibody) | 111.5±23.8 | 7.8 | <0.001 |
| **G3** | KJ015-H (12.5mg/kg) | 119.4±36.8 | 8.4 | <0.001 |
| **G4** | KJ015-H (25mg/kg) | 85.6±30.9 | 6.0 | <0.001 |
| **G5** | KJ015-H (50mg/kg) | 78.9±25.1 | 5.5 | <0.001 |

### Example 10. Detection of Efficacy of KJ015-H Subcutaneous Injection in Combination with αPD-1 Antibody in Treatment of Tumors with High HER2 Expression

The efficacy of KJ015-H subcutaneous injection or trastuzumab in combination with an anti-PD-1 antibody (pembrolizumab, trade name: Keytruda^{®}, manufacturer: Merck) in the treatment of tumors was tested using humanized NOG mice bearing the NCI-N87 model. 3 days before tumor cell inoculation (d-3), 3 × 10⁶ human PBMCs were injected into NOG mice via the tail vein; the day of tumor cell implantation was set as d0; on day 2 after tumor cell inoculation (d1), the mice in groups G3 and G4 began to receive anti-PD-1 antibody treatment at 10 mg/kg, iv, BIW. After the mean tumor volume reached 100 mm³ (day 4 after tumor cell inoculation (d3)), the NOG mice were treated with a control group (normal saline, iv, G1), KJ015-H subcutaneous injection (4 mg/kg, sc, G2), trastuzumab + anti-PD-1 mAb (4+10 mg/kg, iv + iv, G3), or KJ015-H subcutaneous injection + anti-PD-1 mAb (4+10 mg/kg, sc + iv, G4). The tumor volume was measured twice weekly, and the tumors were removed on day 19 after tumor cell inoculation (d18).

The results show that, compared to the control group, the combined use of αPD-1 and KJ015-H subcutaneous injection or trastuzumab could inhibit the growth of HER2-positive NCI-N87 tumors without causing significant body weight loss. Compared to the combined use of trastuzumab and αPD-1, the combined use of KJ015-H subcutaneous injection and αPD-1 had stronger synergistic anti-tumor activity (see FIGs. 7a and 7b).

### Example 11

SK-BR-3 cells were isolated, suspended in an RPMI complete medium, and treated with 200 nM KJ015-H or trastuzumab + pertuzumab (100+100 nM) at 37 °C for 15 min. The antibodies were pre-labeled with Cy5 using a Lightning-Link^{®} labeling kit (ab188288), and each antibody molecule was labeled with an average of 2.5 AF647 molecules. After fixation with 2% phosphoric acid (PFA) in PBS for 10 min at room temperature, the cells were washed with PBS, pelleted on a 0.01% poly-L-lysine-coated cap, and fixed with 2% PFA in PBS. Fiducial markers (ThermoFisher Scientific, Waltham, MA, USA) were used, the cells were washed thoroughly with PBS and pelleted overnight at 4 °C, and unbound beads were removed. Adherent particles were imaged by stochastic optical reconstruction microscopy (iSTORM) (Ningbo INVIEW Technology Co., Ltd., China). Data were processed using the instrument software, the average area of Cy5 fluorescence signals was statistically analyzed, and their distribution was represented as violin plots.

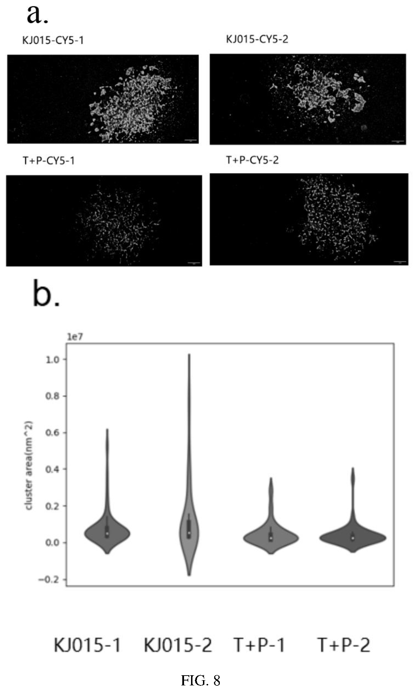

When Cy5-labeled KJ015-H or T+P was investigated in SK-BR-3 cells, high aggregation signal distributions were observed to varying degrees (FIG. 8). The figure indicates that KJ015-H induced a significantly more intense aggregation effect than T+P.

### Example 12

The expression levels of multiple signaling pathways in different tumor cells after incubation with different antibodies (human IgG1, trastuzumab (T), pertuzumab (P), trastuzumab plus pertuzumab (T+P), KJ015-H, anbenitamab (Anbe), zanidatamab (Zani)) were detected by immunoblotting assay.

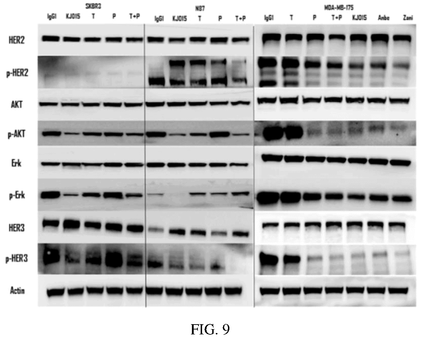

In the immunoblotting results, the expression levels of HER2 and HER3 showed no change, while downstream MAPK signaling was reduced (FIG. 9). It can be reasonably inferred that aggregation reduced the level of normal HER2 heterodimers, preventing the downstream signals from being activated through the normal dimeric signaling pathway. It can be inferred that aggregation may promote endocytosis and impede downstream signaling pathways, thereby reducing tumor cell activity and inhibiting proliferation.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. An anti-HER2 bispecific antibody, comprising a first protein functional region and a second protein functional region for recognizing extracellular domain II and extracellular domain IV of HER2, respectively, wherein the first protein functional region comprises a first heavy chain and a common light chain, and the second protein functional region comprises a second heavy chain and the common light chain; the bispecific antibody remains stable after storage in a PBS buffer at 37±0.5 °C for 30 days.

2. The anti-HER2 bispecific antibody according to claim 1, wherein the common light chain comprises a light chain variable region having the amino acid sequence set forth in SEQ ID NO: 1, the first heavy chain comprises a first heavy chain variable region having the amino acid sequence set forth in SEQ ID NO: 2 or a variant thereof, and the second heavy chain comprises a second heavy chain variable region having the amino acid sequence set forth in SEQ ID NO: 5 or a variant thereof.

3. The bispecific antibody according to claim 1 or 2, further comprising a light chain constant region, wherein the light chain constant region is optionally a human light chain constant region λ chain or κ chain; optionally, an amino acid sequence of the light chain constant region is set forth in SEQ ID NO: 6.

4. The bispecific antibody according to any one of claims 1-3, wherein the first heavy chain and the second heavy chain of the bispecific antibody further comprise heavy chain constant regions, wherein the heavy chain constant regions are optionally human heavy chain constant regions; optionally, a heterodimer is formed between the first heavy chain and the second heavy chain of the bispecific antibody, and the first heavy chain and the second heavy chain are optionally linked via a Knob-in-Hole structure; optionally, the heavy chain constant regions of the first heavy chain and the second heavy chain have the amino acid sequences set forth in SEQ ID NO: 7, 9, or 10 and SEQ ID NO: 8, 11, or 12, respectively.

5. The bispecific antibody according to any one of claims 1-4, wherein the bispecific antibody has a reduced fucose modification; optionally, the antibody has no fucose modification.

6. A nucleic acid molecule encoding the bispecific antibody according to any one of claims 1-5.

7. An expression vector, comprising a nucleotide sequence of the nucleic acid molecule according to claim 6.

8. A prokaryotic or eukaryotic cell, comprising the expression vector according to claim 7.

9. A drug conjugate of an anti-HER2 bispecific antibody, wherein the bispecific antibody according to any one of claims 1-5 is covalently coupled to a cytotoxic drug.

10. A pharmaceutical composition, comprising the bispecific antibody according to any one of claims 1-5 or the drug conjugate according to claim 9, wherein optionally, the pharmaceutical composition further comprises a hyaluronidase and/or an anti-PD1 antibody; optionally, the hyaluronidase is rHuPH20 or modified rHuPH20; optionally, the pharmaceutical composition further comprises a pharmaceutically acceptable carrier or excipient.

11. A method for preparing the bispecific antibody according to any one of claims 1-5, comprising the following steps:
(1) constructing nucleotide sequences which include a nucleotide sequence encoding the first heavy chain, a nucleotide sequence encoding the second heavy chain, and a nucleotide sequence encoding the common light chain in the same vector or different vectors; and
(2) expressing the vector(s) constructed in the above step in different host cells or the same host cell.

12. Use of the anti-HER2 bispecific antibody according to any one of claims 1-5, the drug conjugate according to claim 9, and the pharmaceutical composition according to claim 10 in preparing a medicament for treating an HER2-positive tumor, wherein optionally, the HER2-positive tumor is selected from breast cancer, gallbladder carcinoma, gastric cancer, and lung cancer.
